# EUROPEAN PATENT APPLICATION

(11) **EP 2 543 361 A1**
(43) Date of publication of application: **09.01.2013**
(21) Application number: 11305873.9
(22) Date of filing: 07.07.2011
(51) Int. Cl.: A61K 9/16, A61K 9/50, A61K 31/343, A61K 9/20, A61K 9/28

(54) **Sustained release pharmaceutical oral solid dosage forms of dronedarone or one of its pharmaceutically acceptable salts**

(71) Applicant: SANOFI, 75008 Paris (FR)
(72) Inventor: The designation of the inventor has not yet been filed
(74) Representative: Bouron, Estelle

(57) **Abstract**

The instant invention relates to a sustained release pharmaceutical oral solid dosage form comprising a therapeutically effective amount of dronedarone or pharmaceutically acceptable salts thereof adapted to release over a predetermined time period according to a profile of dissolution characterized in that the total amount of dronedarone or pharmaceutically acceptable salts thereof is liberated at 240 minutes.

Preparation and application in therapeutics.

## Description

The instant invention relates to sustained release pharmaceutical oral solid dosage forms of dronedarone or one of its pharmaceutically acceptable salts, to their preparation and to their application in therapeutics.

In recent years, the clinical utility of drugs can be improved with sustained-release formulation by means of improved therapeutics effect, reduced incidence of adverse effects and simplified dosing regimens.

2-n-Butyl-3-[4-(3-di-n-butylaminopropoxy)benzoyl]-5-methylsulphonamidobenzofuran, or dronedarone, and pharmaceutically acceptable salts thereof are described in European Patent EP 0 471 609 B1.

Dronedarone blocks potassium, sodium and calcium channels and also has anti-adrenergic properties.

Dronedarone is an anti-arrhythmic that is effective in maintaining sinus rhythm to prevent recurrence of atrial fibrillation or to lower ventricular rate as well as prevention of cardiovascular hospitalizations or death in patients presenting atrial fibrillation or atrial flutter.

Currently, a dosage regimen of dronedarone of 400 mg in base form equivalent twice a day is employed using immediate release tablets. This may be undesirable at least because patient compliance decreases as the frequency of taking a drug increases.

There is therefore an existing need for a once a day modified release formulation comprising dronedarone or one of its pharmaceutically acceptable salts with reliable slower absorption over a targeted period of time.

Thus a subject of the invention is a sustained release pharmaceutical oral solid dosage form comprising a therapeutically effective amount of dronedarone or pharmaceutically acceptable salts thereof adapted to release over a predetermined time period according to a profile of dissolution characterized in that the total amount of dronedarone or pharmaceutically acceptable salts thereof is liberated at 240 minutes.

Another subject of the invention is a sustained release pharmaceutical oral solid dosage form comprising a therapeutically effective amount of dronedarone or pharmaceutically acceptable salts thereof adapted to release over a predetermined time period according to a profile of dissolution characterized in that the total amount of dronedarone or pharmaceutically acceptable salts thereof is liberated at 240 minutes, said dissolution profile is measured after introducing said sustained release pharmaceutical oral solid dosage form in a solution at pH = 4,5 comprising potassium dihydrogen orthophosphate in 1000 ml of water.

Another subject of the invention is a sustained release pharmaceutical oral solid dosage form comprising a therapeutically effective amount of dronedarone or pharmaceutically acceptable salts thereof adapted to release over a predetermined time period according to a profile of dissolution characterized in that 18 to 35 % of dronedarone or pharmaceutically acceptable salts thereof is liberated at 30 minutes and the total amount of dronedarone or pharmaceutically acceptable salts thereof is liberated at 240 minutes.

Another subject of the invention is a sustained release pharmaceutical oral solid dosage form comprising a therapeutically effective amount of dronedarone or pharmaceutically acceptable salts thereof adapted to release over a predetermined time period according to a profile of dissolution characterized in that 30% of dronedarone or pharmaceutically acceptable salts thereof is liberated at 30 minutes and 70% of dronedarone or pharmaceutically acceptable salts thereof is liberated at 240 minutes.

Another subject of the invention is a sustained release pharmaceutical oral solid dosage form comprising a therapeutically effective amount of dronedarone or pharmaceutically acceptable salts thereof adapted to release over a predetermined time period according to a profile of dissolution characterized in that the total amount of dronedarone or pharmaceutically acceptable salts thereof is liberated at 240 minutes and in that it comprises a release controlling agent.

Another subject of the invention is a sustained release pharmaceutical oral solid dosage form comprising a therapeutically effective amount of dronedarone or pharmaceutically acceptable salts thereof adapted to release over a predetermined time period according to a profile of dissolution characterized in that 18 to 35 % of dronedarone or pharmaceutically acceptable salts thereof is liberated at 30 minutes and the total amount of dronedarone or pharmaceutically acceptable salts thereof is liberated at 240 minutes and in that it comprises a release controlling agent.

Another subject of the invention is a sustained release pharmaceutical oral solid dosage form comprising a therapeutically effective amount of dronedarone or pharmaceutically acceptable salts thereof adapted to release over a predetermined time period according to a profile of dissolution characterized in that 30% of dronedarone or pharmaceutically acceptable salts thereof is liberated at 30 minutes and 70% of dronedarone or pharmaceutically acceptable salts thereof is liberated at 240 minutes and in that it comprises a release controlling agent.

Said release controlling agent may be selected from the group comprising polymers of N-vinyl-2-pyrrolidone such as crospovidone, particularly in a range between 1% and 10% of the total amount of dosage form, and more particularly in a range between 2% and 6% of the total amount of dosage form.

Said sustained-release pharmaceutical oral solid dosage form may also comprise a pharmaceutically acceptable non ionic hydrophilic surfactant such as polyoxyethylene-polyoxypropylene copolymers also called poloxamers, more particularly poloxamer 407 particularly in a range between 1% to 20% of the amount of dronedarone as base form, particularly in a range between 5% to 15% of the amount of dronedarone as base form and more particularly at 10% of the amount of dronedarone as base form and/or in a range between 7.5 and 9 % of the total amount of dosage form and more particularly around 8% of the total amount of dosage form.

Among the pharmaceutically acceptable salts of dronedarone, mention may be made of the hydrochloride.

The sustained-release dronedarone compositions may be matrix formulations.

More specifically, crospovidone may be used in a matrix formulation.

In another embodiment, the invention provides a sustained release pharmaceutical oral solid dosage form as described above which is an eroding matrix formulation, wherein the matrix material is selected from the group comprising polyoxyethylene-polyoxypropylene copolymers also called poloxamers, more particularly poloxamer 407 particularly in a range as mentioned above and polymers of N-vinyl-2-pyrrolidone such as crospovidone, particularly in a range between 1% and 10% and more particularly in a range between 2% and 6% of the total amount of dosage form..

Said sustained release pharmaceutical oral solid dosage form may be monolithic, such as tablets.

The sustained release pharmaceutical oral solid dosage form according to the invention may also comprise others excipients for example glidant such as colloidal anhydrous silica, lubricant such as magnesium stearate, coating agent -opacifier-pigment such as titanium oxide, plasticizer such as Macrogol 6000, coating agent such as carnauba wax.

Said excipients are chosen according to the pharmaceutical form and the administration route desired, among usual excipients known of one of skill in the art.

The sustained release pharmaceutical oral solid dosage form according to the invention can be prepared by use of well known pharmaceutical techniques such as blending, granulation, milling, tabletting or coating.

List of figures:
Figure 1 shows dissolutions profiles of current dronedarone 400 mg immediate release tablet;
Figure 2 shows dissolutions profiles of examples according to the invention.

The following examples describe the synthesis of some dosage forms according to the invention. These examples are not intended to be limitative and only illustrate the present invention.

To examplify the interest of proposed pharmaceutical compositions in the obtention of a form containing a high dose of dronedarone (i.e. 800mg) allowing a once a day administration, various examples are presented hereafter.

### EXAMPLE: Manufacture of monolithic tablets

### 1.1 Granules A

Formulations were made from the following basis: first 9.140 kg of dronedarone hydrochloride and 0.860 kg of poloxamer 407 are blended manually. The blended material is screened through a 1.5 mm screen using a conical screening mill rotating at 530 rpm. The screened material is blended in a bin-blender for 30 minutes at 7 rpm to produce a homogeneous blend.

Then, the blend is granulated using a double screw equipment fitted with a double wall using the following parameters: blend feeding 5 kg/h, rotating speed of the screws 200 rpm, temperature of the screws 86.1 to 90.3°C, granulation couple from 5 to 12 N.m. The granulated material is collected on trays to allow its cooling. This granulated base will be called granule A for next steps.

| Granules A | |
|---|---|
| Components | Amount (mg) |
| Dronedarone hydrocloride | 852 |
| Poloxamer | 80 |

### 1.2 Formulations B and B'

1398 g of granules A are screened with 11.25 g of colloidal anhydrous silica and 14.25 g of magnesium stearate through a 1.5 mm screen using a conical screening mill rotating at 530 rpm. Screened material is then blended manually.

Then, 19x10 mm oblong shape tablets, each comprising 800 mg of dronedarone hydrochloride are compressed on a Kilian S100 press under a pressure of 15 kN. The uncoated tablets are called **Formulation B.**

Then a coating suspension is prepared by dispersing 65.25 g of hydroxypropyl methyl cellulose 6 mPa.s, 9 g of titanium dioxide, 15.75 g of macrogol 6000 in 765 g of purified water. Uncoated tablets are coated in a side vented coating pan MiniGlatt until an increase of mass of 15 mg. Then slight amount of carnauba wax is added to the coated tablets in order to improve gliding properties. The finished coated tablets are called **Formulation B'.** Mass of the finished product = 964 mg.

| Formulation B and B' | |
|---|---|
| Components | Amount (mg) |
| Dronedarone hydrochloride | 852 |
| Poloxamer | 80 |
| Colloidal anhydrous silica | 7.5 |
| Magnesium stearate | 9.5 |
| Uncoated tablet / Formulation B | 949 |
| Hydroxypropyl methyl cellulose 6 mPa.s | 10.875 |
| Titanium dioxide | 1.5 |
| Macrogol 6000 | 2.625 |
| Carnauba wax | 0.195 |
| Coated tablet / / Formulation B' | 964 |

### 1.3 Formulation C

512.6 g of granules A are screened with 55 g of crospovidone, 2.2 g of colloidal anhydrous silica and 2.75 g of magnesium stearate through a 1.5 mm screen using a conical screening mill rotating at 530 rpm. Screened material is then blended manually. This blend is called **blend c.** Then, 19x10 mm oblong shape tablets, each comprising 800 mg of dronedarone hydrochloride are compressed on a Kilian S100 press under a pressure of 11 kN. The uncoated tablets are called **Formulation C.** Mass of the finished product = 1041 mg.

| Formulation C | |
|---|---|
| Components | Amount (mg) |
| Dronedarone hydrochloride | 852 |
| Poloxamer | 80 |
| Colloidal anhydrous silica | 4 |
| Magnesium stearate | 5 |
| Crospovidone | 100 |
| Uncoated tablet / Formulation C | 1041 |

### 1.4 Formulation D

186.4 g of granule A is screened with 0.8 g of colloidal anhydrous silica and 1.0 g of magnesium stearate through a 1.5 mm screen using a conical screening mill rotating at 530 rpm. Screened material is then blended manually with 208.2 g of blend c (see 1.3).

Then, 19x10 mm oblong shape tablets, each comprising 800 mg of dronedarone hydrochloride are compressed on a Kilian S100 press under a pressure of 10 kN. The uncoated tablets are called **Formulation D.** Mass of the finished product = 991 mg.

| **Formulation D** | |
|---|---|
| Components | Amount (mg) |
| Dronedarone hydrochloride | 852 |
| Poloxamer | 80 |
| Colloidal anhydrous silica | 4 |
| Magnesium stearate | 5 |
| Crospovidone | 50 |
| Uncoated tablet / **Formulation D** | 991 |

### 1.5 Formulation E

466 g of granules A are screened with 12.5 g of crospovidone,2.0 g of colloidal anhydrous silica and 2.5 g of magnesium stearate through a 1.5 mm screen using a conical screening mill rotating at 530 rpm. Screened material is then blended manually.This blend is called **blend e.** Then, 19x10 mm oblong shape tablets, each comprising 800 mg of dronedarone hydrochloride are compressed on a Kilian S100 press under a pressure of 10 kN. The uncoated tablets are called **Formulation E.** Mass of the finished product = 966 mg.

| **Formulation E** | |
|---|---|
| Components | Amount (mg) |
| Dronedarone hydrochloride | 852 |
| Poloxamer | 80 |
| Colloidal anhydrous silica | 4 |
| Magnesium stearate | 5 |
| Crospovidone | 25 |
| Uncoated tablet / Formulation E | 966 |

### 1.7 Formulations G and G'

932 g of granules F is screened with 12.5 g of crospovidone, 3.8 g of colloidal anhydrous silica and 4.7 g of magnesium stearate through a .1.5 mm screen using a conical screening mill rotating at 530 rpm. Screened material is then blended manually.This blend is called **blend g.**

Then, 19x10 mm oblong shape tablets, each comprising 800 mg of dronedarone hydrochloride are compressed on a Kilian S100 press under a pressure of 10 kN. The uncoated tablets are called **Formulation G.**

Then a coating suspension is prepared by dispersing 54.375 g of hydroxypropyl methyl cellulose 6 mPa.s, 7.5 g of titanium dioxide and 13.125 g of macrogol 6000 in 637.5 g of purified water. Uncoated tablets **Formulation G** are coated in a side vented coating pan MiniGlatt until a increase of mass of 15 mg. The finished coated tablets are called **Formulation G'.** Mass of the finished product = 968 mg.

| Formulation G and G' | |
|---|---|
| Components | Amount (mg) |
| Dronedarone hydrochloride | 852 |
| Poloxamer | 80 |
| Colloidal anhydrous silica | 3.8 |
| Magnesium stearate | 4.7 |
| Crospovidone | 12.5 |
| Uncoated tablet / **Formulation G** | 953 |
| Hydroxypropyl methyl cellulose 6 mPa.s | 10.875 |
| Titanium dioxide | 1.5 |
| Macrogol 6000 | 2.625 |
| Coated tablet / **Formulation G'** | 968 |

Table 1 summarizes unit formula tested for monolithic forms

**Table 1 Monolithic forms examples - Unit formula**

| Example ref | B' | D | E | G' |
|---|---|---|---|---|
| Components (mg) | | | | |
| Dronedarone hydrochloride | 852 | 852 | 852 | 852 |
| Poloxamer | 80 | 80 | 80 | 80 |
| Colloidal anhydrous silica | 7.5 | 4 | 4 | 3.8 |
| Magnesium stearate | 9.5 | 5 | 5 | 4.7 |
| Crospovidone | 0 | 50 | 25 | 12.5 |
| Uncoated tablet mass | 949 | 991 | 966 | 953 |
| Hydroxypropyl methyl cellulose 6 mPa.s | 10.875 | 0 | 0 | 10.875 |
| Titanium dioxide | 1.5 | 0 | 0 | 1.5 |
| Macrogol 6000 | 2.625 | 0 | 0 | 2.625 |
| Carnauba wax | 0.195 | 0 | 0 | 0 |
| Coated tablet mass | 964 | | | 968 |

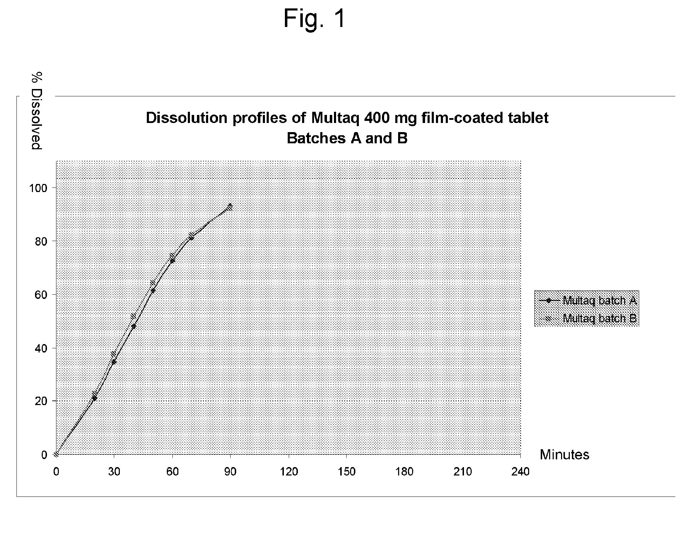

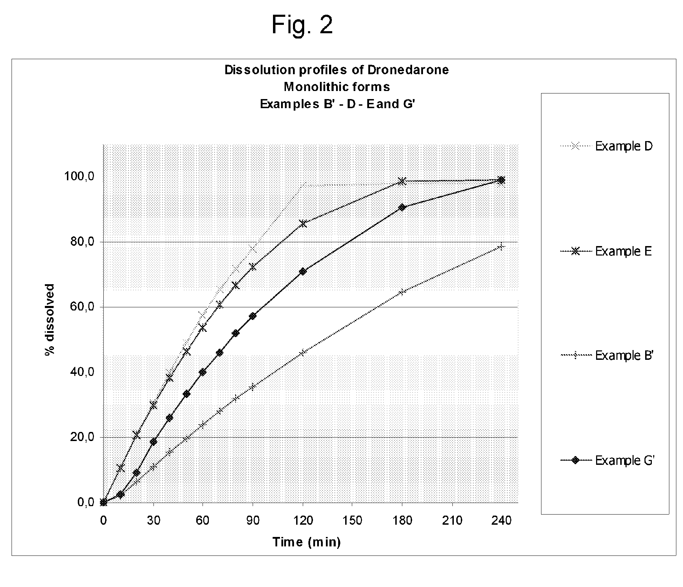

Dissolution profiles of immediate release tablet and examples using dissolution methods described hereafter are presented in figures 1 and 2.

### Dissolution method description for current dronedarone 400 mg immediate release tablet

This dissolution method proceed according to the dissolution test for solid oral dosage forms (Ph. Eur. 2.9.3 and USP <711>) using a paddle apparatus.

The dissolution medium is prepared by dissolving 13.61 mg of potassium dihydrogen orthophosphate in 1000 ml of ultra-purified water. If necessary, adjust the pH to 4.50 ± 0.05 with 0.1M hydrochloric acid or 0.1M sodium hydroxide.

The experimental conditions are:
- Introduce 1000 ± 10 ml of dissolution medium into each vessel
- Set the temperature to 37 ± 0.5°C and the paddle speed at 75 rpm.

The reference solution is prepared by dissolving 42.6 mg of dronedarone hydrochloride working standard in 2.0 ml of methanol and dilute to 100.0 ml with dissolution medium.

The procedure comprises the following steps:
- Introduce one tablet, into each of six dissolution vessels and immediately start the paddles.
- Using an automatic system, sample the medium at 10, 20, 30, 40, 50, 60, 70, 80 and 90 minutes.
- Filter the samples through glass filters 10µm (Whatman GF/D, or equivalent) or polypropylene filters (Prolabo, or equivalent).
- Determine the dronedarone hydrochloride spectrophometrically at 288 nm using a 1 mm quartz cell and dissolution medium as the blank.
- Measure the absorbance of the reference solution under the same conditions.

### Dissolution method description for the examples

This dissolution method proceed according to the dissolution test for solid oral dosage forms (Ph. Eur. 2.9.3 and USP <711>) using a paddle apparatus.

The dissolution medium is prepared by dissolving 13.61 mg of potassium dihydrogen orthophosphate in 1000 ml of ultra-purified water. If necessary, adjust the pH to 4.50 ± 0.05 with 0.1 M hydrochloric acid or 0.1M sodium hydroxide.

The experimental conditions are:
- Introduce 1000 ± 10 ml of dissolution medium into each vessel
- Set the temperature to 37 ± 0.5°C and the paddle speed at 75 rpm.

The reference solution is prepared by dissolving 85.2 mg of dronedarone hydrochloride working standard in 2.0 ml of methanol and dilute to 100.0 ml with dissolution medium.

The procedure comprises the following steps:
- Introduce one tablet, into each of three dissolution vessels and immediately start the paddles.
- Using an automatic system, sample the medium at 10, 20, 30, 40, 50, 60, 70, 80, 90, 120, 180 and 240 minutes.
- Filter the samples through glass filters 10µm (Whatman GF/D, or equivalent) or polypropylene filters (Prolabo, or equivalent).
- Determine the dronedarone hydrochloride spectrophometrically at 264 nm using a 1 mm quartz cell and dissolution medium as the blank.
- Measure the absorbance of the reference solution under the same conditions.

Dissolution profiles in Figures 1 and 2 clearly show that formulations according to the invention are sustained release formulations.

In the sustained release pharmaceutical oral solid dosage form according to the invention, dronedarone or a pharmaceutically salt thereof can be administered as a unitary dosage form, in blend with usual pharmaceutical excipients, to animals and human beings for the prevention or for the treatment of diseases mentioned above.

By the oral route, the dose of active principle to administer per day can reach 800 mg.

In specific cases, higher or lower dosages may be appropriate; these dosages are comprised within the scope of the present invention. According to usual practice, the dosage suitable to each patient is determined by the physician according to the administration route, the weight and response of the patient.

The present invention, according to another of its aspects, also relates to a method for the treatment of the above pathologies, which comprises the administration to a patient of an effective dose of a dronedarone or a salt with a pharmaceutically acceptable salt thereof.

## Claims

1. Sustained release pharmaceutical oral solid dosage form comprising a therapeutically effective amount of dronedarone or pharmaceutically acceptable salts thereof adapted to release over a predetermined time period according to a profile of dissolution **characterized in that** the total amount of dronedarone or pharmaceutically acceptable salts thereof is liberated at 240 minutes.

2. Dosage form according to claim 1 **characterized in that** said dosage form comprises a release controlling agent which is selected from the group comprising polymers of N-vinyl-2-pyrrolidone.

3. Dosage form according to claim 2 **characterized in that** said polymers of N-vinyl-2-pyrrolidone is crospovidone.

4. Dosage form according to anyone of the preceding claims **characterized in that** it also comprises a pharmaceutically acceptable non ionic hydrophilic surfactant.

5. Dosage form according to claim 4 **characterized in that** said pharmaceutically acceptable non ionic hydrophilic surfactant is polyoxyethylene-polyoxypropylene copolymers also called poloxamers.

6. Dosage form according to anyone of the preceding claims **characterized in that** pharmaceutically acceptable salts of dronedarone is the hydrochloride.

7. Dosage form according to anyone of the preceding claims **characterized in that** said dosage form is a matrix formulation.

8. Dosage form according to anyone of the preceding claims **characterized in that** said sustained release pharmaceutical oral solid dosage form is monolithic, such as tablets.

9. Dosage form according to anyone of the preceding claims **characterized in that** said sustained release pharmaceutical oral solid dosage form is an eroding matrix formulation, wherein the matrix material is selected from the group comprising polyoxyethylene-polyoxypropylene copolymers and polymer of N-vinyl-2-pyrrolidone.

10. Medicament, comprising a dosage form according to claims 1 to 9.

11. Dosage form according to claims 1 to 9 for use in the treatment of atrial fibrillation or the prevention of recurrence of atrial fibrillation or death or cardiovascular hospitalization.
